Europäisches Patentamt

European Patent Office

Office européen des brevets

(19)

(11) Publication number: **0 189 656 B1**

(12) **EUROPEAN PATENT SPECIFICATION**

(45) Date of publication of patent specification: **12.06.91** (51) Int. Cl.⁵: **B01J 13/02**

(21) Application number: **85308926.6**

(22) Date of filing: **09.12.85**

(54) On page fragrance sampling device.

(30) Priority: **21.12.84 US 684597**

(43) Date of publication of application:
**06.08.86 Bulletin 86/32**

(45) Publication of the grant of the patent:
**12.06.91 Bulletin 91/24**

(84) Designated Contracting States:
**DE FR GB IT SE**

(56) References cited:
**US-A- 4 186 743**
**US-A- 4 487 801**

(73) Proprietor: **MINNESOTA MINING AND MANU-FACTURING COMPANY**
**3M Center, P.O. Box 33427**
**St. Paul, Minnesota 55133-3427(US)**

(72) Inventor: **Charbonneau, Jack W. c/o Minnesota Mining and**
**Manufacturing Company 2501 Hudson Road**
**St. Paul Minnesota 55133-3427(US)**
Inventor: **Relyea, Keith E. c/o Minnesota Mining and**
**Manufacturing Company 2501 Hudson Road**
**St. Paul Minnesota 55133-3427(US)**

(74) Representative: **Baillie, Iain Cameron et al**
**c/o Ladas & Parry Isartorplatz 5**
**W-8000 München 2(DE)**

Rank Xerox (UK) Business Services

## Description

Field of the Invention

This invention relates to microencapsulated materials, articles containing microencapsulated materials and the method of preparing such articles. In particular, the present invention relates to labels of microencapsulated materials adhesively secured between two temporarily adhered surfaces such that upon adhesion of the label and separation of said two surfaces, the capsules rupture, releasing material contained therein.

Background of the Invention

Encapsulated materials have been used for many years in a wide variety of commercial applications. Early uses of encapsulated materials included paper coated with capsules bearing coloring material therein which could be used as a recording medium. U.S. Patent No. 3,016,308 discloses one of the early efforts using encapsulated material as the image source on recording paper. U.S. Patent Nos. 4,058,434 and 4,201,404 show other methods of application of encapsulated coloring materials on paper substrates to be used as imaging media and the like. U.S. Patent No. 3,503,783 shows microcapsules having coloring material therein which are ruptureable by the application of heat, pressure and/or radiation because of a metal coating on the surface of the capsule. These ruptureable microcapsules, in one embodiment, may be secured between a substrate and a photoconductive top coat to enable photosensitive imaging of the system.

A wide variety of processes exist by which microcapsules can be manufactured. These varied processes provide different techniques for producing capsules of varying sizes, alternative materials for the composition of the capsule shell and various different functional materials within the shell. Some of these various processes are shown in U.S. Patent Nos. 3,516,846; 3,516,941; and British Patent Specification Nos. 1,156,725; 2,041,319 and 2,048,206. A wide variety of different materials may also be used in making the capsule shells. A popular material for shell formation is the polymerization reaction product between urea and formaldehyde or melamine and formaldehyde, or the polycondensation products of monomeric or low molecular weight polymers of dimethylolurea or methylolated urea with aldehydes. A variety of capsule forming materials are disclosed, for example, in U.S. Patent Nos. 3,516,846 and 4,087,376 and U.K. Patent Specification Nos. 2,006,709 and 2,062,570.

As shown in these references, the principal utility of microencapsulated materials is in the formation of a surface coated with the microcapsules in a binder. The microcapsules are ruptured by various means to release the material contained therein. In addition to release of physically observable materials such as ink in order to form a visible image, other types of active ingredients such as odor releasing materials, bacteriostatic materials, chemically active materials and the like have been provided in this manner.

U.S. Patent 4,186,743 discloses a perfuming self-adhering sanitary napkin having a pressure-sensitive adhesive layer bonded to a strippable cover sheet having a binder layer with microcapsules on the surface thereof in contact with the pressure-sensitive adhesive layer. Upon stripping of the cover sheet, capsules are broken, the pressure sensitive adhesive is exposed and the napkin may adhere to undergarments to keep them properly positioned.

U.S. Patent 4,487,801 discloses a fragrance releasing pull-apart sheet comprising a non-pressure-sensitive binder layer containing microcapsules adhered between two sheets. Upon separation of the sheets, the adhesive and capsules rupture, releasing the material within the capsules.

Pull-apart inserts such as those disclosed in U.S. 4,487,801 are usually saddle-stitched, perfect bound or center-stapled (by staples or glue) into magazines and cost as much as a full page. Even with art work (e.g., printing) on the pull apart sheets, after opening the sheets, tend to be unattractive and do not usually compare with the size and thickness of other pages in the magazines. Certain manufacturers of these inserts lack sufficient expertise to prevent the sheets from opening during transit and the magazines tend to be overwhelmed with a multiplicity of scents, thereby reducing the effectiveness of individual inserts.

US 3,494,505 discloses a tape in a dispenser, a layer of binder material incorporating microcapsules of perfume being provided on one surface of the tape and a layer of adhesive being provided on the other surface of the tape and protected with a removable backing strip. On withdrawing the tape from the dispenser, the microcapsules are crushed and release the perfume. The adhesive enables the tape to be attached to the hem of a garment, for example.

Summary of the Invention

The present invention provides a label for application to a surface, said label comprising

1) at least two sheets or a folded single sheet having opposed, faces, the faces of the sheets being bound by an adhesive composition layer,

2) said adhesive composition layer containing microcapsules with a liquid within the shell of the microcapsules,

3) said microcapsules having an average diameter between 4 and 80 micrometers, and

4) one of said sheet(s) having a further adhesive on an exterior surface.

the cohesive strength of said adhesive composition layer being less than the strength of the bond between said adhesive composition and the opposed faces of said sheets, the tensile rupture strength of said microcapsules being such that the cohesive failure of the adhesive results in breakage of the microcapsules, the tensile rupture strength between said two sheets being at least 0.981 Nm-1 (1.0g/cm) and less than 88.3 Nm-1 (90g/cm) at 20°C and 50% relative humidity, the adhesive strength to printed coated paper said further adhesive on said exterior surface being greater than the cohesive strength of said adhesive composition layer containing microcapsules.

The adhesive composition need not extend across the entire surface of the opposed sheet faces and can thereby provide a region of non-adherence between said sheet faces.

The sheets can be flexible sheets of coated paper and said adhesive composition can comprise said microcapsules, a polymeric binder and a viscofier, and said viscofier comprises from 0.25 to 25% or particularly between 0.25 and 12% by dry weight of said adhesive composition layer.

The microcapsules can comprise gelatin and constitute between 20 and 90% by volume of said adhesive composition, and said binder can comprise between 0 and 78.75% by weight of said adhesive composition. In use, the microcapsules are ruptured by pulling apart the sheets after they are first adhered to another surface which causes the capsules to rupture and release the ingredients contained therein. By selecting the relative physical properties of the sheet, adhesive, capsules and the binding forces amongst them, a high rate of capsule rupturing can be obtained consistently.

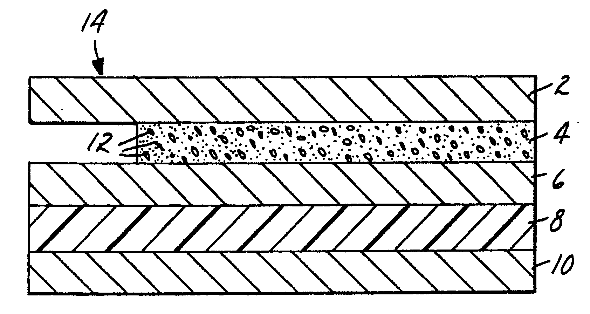

## Summary of the Drawing

The Figure shows a side view of the appliable label of the present invention.

## Detailed Description of the Drawing

The Figure shows the label 14 of the present invention of a top sheet 2 secured to bottom sheet 6 by an adhesive layer 4 having rupturable microcapsules 12 therein. The surface of the bottom sheet 6 which is away from the top sheet 2 has a pressure sensitive adhesive layer 8 thereon. The pressure-sensitive adhesive layer 8 is usually protected before use by a strippable cover sheet 10.

## Detailed Description of the Invention

The sheet material utilised in the label of the present invention may comprise any sheet or film forming material, particularly paper and most preferably coated paper. Generally flexible sheets of paper are preferred although polymeric films may be used. Coated paper is a conventional and standard item in commerce. It is generally a fibrous sheet having a pigment-bearing resinous coating on one or both surfaces. Usually the pigment provides a white, bone or ivory coloration to the sheet. Most generally pigments producing a white coloration are used. The binder used in the resinous coating is generally colorless and/or transparent. The binder is generally a synthetic or natural organic polymeric material. Typical pigments for producing white coated paper are fine white pigment such as clay, calcium carbonate, titania, silica, zinc oxide, etc. Typical binders include latices (e.g., styrene-butadiene, butadiene-acrylonitrile, etc.), film-forming polymers (e.g., polymethylmethacrylate), and natural resins (e.g., casein, ammonium caseinate, starch, etc.). The coatings usually comprise between 65-90% by weight of pigment, preferably 70-80% by weight of pigment, and 10-35% by weight of binder, preferably 20-30% by weight of binder. Papers having both sides coated are preferred in the advertising trade.

The adhesive material for the capsules must form a bond to the coated surfaces of the sheets which is stronger than the cohesive strength of the adhesive with the capsules dispersed therein. Although it is generally desirable to have an adhesive, the absolute cohesive strength of which is less than its adhesive strength to the coated surface of the coated paper cover sheets, this is not essential. When capsules are included within the adhesive composition, the effective cohesive strength of the adhesive tends to be

3

reduced. Adhesives, which by themselves would cause the sheets to be damaged during separation, can be used in combination with capsules in the practice of the present invention because of lowered effective cohesive strength. The capsules in the present invention may comprise any ruptureable capsule containing an active ingredient therein. The tensile rupture strength of the capsules must be such that the cohesive failure of the adhesive results in capsule breakage. It has also been found that the size of the capsules plays a role in the usefulness of capsules within ruptureable sheets according to the practice of the present invention. Desirably the capsules should have an average diameter between 6 and 50 $\mu$m and preferably between 12 and 30 $\mu$m when the capsule concentration is between 80 and 90% by weight of the total capsule weight. It is highly preferred that the capsules have an average diameter between 14 and 26$\mu$m and it is most preferred that the capsules have a diameter between 15 and 25 $\mu$m. These dimensions play a surprisingly important role in the ability to control the percentage of rupture of capsules in the practice of the present invention. With lower concentrations (e.g. 70-80%), the capsules should be larger to provide the necessary rupture strength. The broadest range of capsule size under any conditions would be about 4 to 80 $\mu$m, with 8 $\mu$m capsules used with a 90-95% by weight concentration. Eight to thirty $\mu$m capsules are generally preferred.

Any adhesive capable of bonding the label to another surface may be used in the exterior surface of one of said sheets. Typically, acrylate and polyurethane pressure-sensitive adhesives are used to bond the article to another surface. Thermally sofenable adhesives, such as polyolefins, polyamides, and polyesters are also particularly useful. Solvent-activatable adhesives (including water-activatable adhesives) such as poly(vinyl alcohol), natural gums, acrylates and polyesters are also very useful. Thus pressure-sensitive, heat activatable, or solvent activatable adhesive can be used, as well as permanent adhesives coated on the exterior surface immediately before application of the sampling device.

A basic relationship exists amongst the factors of peel force adhesive coating weight and the median capsule diameter. This relationship can be expressed as $P = k\ (^{Cw}/d^2)$, wherein P equals the peel force, $C^w$ equals the adhesive line coating weight, d equals the median diameter of the capsules and k equals a co-efficient relating to binder and substrate properties. The peel force should be in the range of 16.4 $Nm^{-1}$ to 131 $Nm^{-1}$ (1.5 to 12 ounces per linear inch), preferably 16.4 $Nm^{-1}$ to 87.6 $Nm^{-1}$ (1.5 to 8.0 ounces per linear inch). The coating weight of adhesive and microcapsules should be at a coating weight of approximately 1 kg per 61.4 to 163.8m$^2$ (one pound for 300 to 800 square feet). Preferably the coating weight should be between approximately 1 kg per 81.9 to 133.1m$^2$ (one pound for each 400 to 650 square feet). At higher coating weights, the surface of the cover sheets tend to tear, while at lower coating weights, the sheets tend to pull apart and the adhesive to paper bond tends to rupture in advance of the capsules included therein. The capsules should form between 20 and 90 percent by volume of the total adhesive composition, and preferably between 50 and 85 percent of the total composition volume. If certain microcapsule shell materials are used, such as gelatin, the capsule may comprise as much as 100% of the adhesive compositions.

There are numerous advantages to the practice of the present invention and techniques for improving products using the present invention. The cost of the label inserts of the present invention is far less than the existing inserts which are equivalent to adding an additional page. The insertion of the label inserts of the present invention can be done with existing label application equipment. The label inserts can be preprinted before application. Perforated lines can be cut into the exposed surface so that multiple uses of the pull apart sheet are available, with each strip being pulled off separately. The artwork on the applied label may be the same as or different from the artwork over which it is applied. For example, the artwork over the label may show an unopened flower bud and the artwork under the label which is exposed upon removal of the top sheet and release of the fragrance may show an opened flower, thus providing a visual change as well as a change in scents. Similarly, the label could show the top of a perfume bottle with the stopper in it while the artwork underneath could show an open bottle. The residual, unbroken capsules could still be broken by scratching the surface, providing continued utility for the page after removal of the top sheet. Using labels, according to the present invention, it is also much simpler to apply multiple fragrances to a single page by applying more than one label with each label having a different fragrance.

The rupture force of the adhesive composition layer containing microcapsules at 50% relative humidity is between at least 1 ounce per linear five-and-one-half inches and less than 45 ounces per linear five-and-one-half inches (greater than 1.96 $Nm^{-1}$ (2.0g/cm) and less than 88.3 $Nm^{-1}$ (90g/cm)). It is preferred that the rupture strength between the sheets exceeds 7.85 $Nm^{-1}$ (8.0g/cm) and is less than 78.5 $Nm^{-1}$ (80g/cm) and most preferably exceeds 15.7 $Nm^{-1}$ (16g/cm) and is less than 73.6 $Nm^{-1}$ (75g/cm). The minimum strength at this ambient condition (i.e. 20$^\circ$C and 50% R.H.) is necessary to keep the sheets from falling apart from forces incurred during handling. This problem has frequently occurred in magazine inserts where coated paper was used. The maximum limit on the rupture strength is necessary to keep the paper from tearing (termed fiber pull or fiber rupture) before the adhesive and capsules rupture. This would prevent release of the liquid from the capsules. The adhesive strength of the exterior adhesive to printed coated paper should be greater than the cohesive strength of the adhesive layer containing microcapsules. Preferably, it is at least 10% greater in adherence than the rupture strength between the sheets. This type of article is generally applied to paper surfaces (and especially coated paper surfaces) and so it is preferred that the strength of adherence of the exterior adhesive to paper is at least 10% greater than the rupture strength between the sheets. A "liquid" according to the present invention includes liquids with materials dissolved or dispersed therein (e.g., pigments) and gels which are capable of flowing under moderate pressure.

It is also desirable to have the construction resist the effects of variable ambient conditions. It is therefore desirable that rupture strength excede 3.9 Nm⁻¹ (4.0g/cm) after storage at 120°C and less than 1% R.H. for seventy-two hours. This test would be performed by storage in an oven, removal to a neutral environment (e.g., sealed bag or jar) until the article is at room temperature, and then measuring the rupture strength. It is preferred that the rupture strength is at least 7.85 Nm⁻¹ (8.0g/cm) and most preferred that the rupture strength is at least 15.7 Nm⁻¹ (16g/cm) under those conditions. The label should preferably still display a rupture strength between 1.96 Nm⁻¹ and 88.3 Nm⁻¹ (2 and 90g/cm) at 20°C and 50% R.H.

A number of methods have been found which enable these conditions to be met according to the present invention. The rise of viscosity increasing agents in the binder provides a more even coating and one that ruptures before fiber pull begins. The use of additional coatings over the coated paper which contain polymers different from the binder of the adhesive layer and which do not form a solution or chemically bond to the binder of the adhesive layer provides a useful article according to the present invention. The use of larger size capsules tends to weaken the cohesive strength of the adhesive composite and prevent fiber pull. The use of capsules which are not moisture sensitive in combination with these large capsules (i.e., greater than 30 μm and up to 95 μm) provides a useful adhesive layer. Higher capsule-to-binder ratios reduce the cohesive strength of the adhesive, as does the addition of non-viscosity enhancing particulate fillers.

Preferably, if the label utilises coated paper surfaces, the binder between the sheets contains viscosity increasers (viscofiers) in addition to the microcapsules. The use of viscofiers reduces the criticality of proportions of materials and provided increased coating and manufacturing latitude. Viscosity enhancers or viscosity increasing agents are well known in the art. Any material which when present in the coating solution in an amount not greater than 10% by weight increases the viscosity by at least 5% is a viscofier according to the present invention. Preferably viscosity is increased by at least 20%. Suitable materials include either inorganic particulate materials (e.g., silica, amorphous silica, bentonite clay, montmorillonite clay, etc.) or organic particulate or soluble materials (e.g., water softenable acrylic particles, water swellable poly(methylmethacrylate), water soluble or organic solvent soluble polymers, etc.). The inorganic particles tend to be preferred. The viscofiers enhancers have been found to be necessary in dry weight proportions of the adhesive mix in amounts of from 0.25 to 12% by weight, preferably from 5 to 12% by weight. In general, the weight proportions of materials in the dried adhesive layers according to the present invention are generally as follows:

| | | |
|---|---|---|
| Microcapsules | 21 | – 80% |
| Adhesive | 19.75 | – 70% |
| Viscosity Enhancers | 0.25 | – 12% |

Other optional ingredients such as surfactants, coating aids and the like may be present. Preferred proportions of these ingredients are:

| | | |
|---|---|---|
| Microcapsules | 44.5 | – 80% |
| Adhesive | 19.5 | – 55% |
| Viscosity Enhancers | 0.5 | – 10% |

The ability to use coated paper in the manufacture of these articles is important because those materials are the standard printing medium of the trade. Both one-side coated paper and two-side coated paper are useful. Those papers enable the highest quality printings to be made in combination with the

5

releasable materials of the present invention.

The nature and composition of the adhesive binder is not critical to the practice of the invention as long as the required adhesive and cohesive properties are met. The adhesive may be pressure sensitive, water or solvent soluble or thermally activatable. A single layer of a non-pressure-sensitive adhesive is preferred. There is no need for rejoining the sheets after rupturing of the capsules and so the pressure sensitive function is not necessary.

The adhesive (with microcapsules) may be applied between two separate sheets in either a continuous or discontinuous patterns. It is usually desirable to leave at least some portion of at least one outer edge of the sheets unbonded so as to provide an area where separation can be easily started. A single sheet may be folded so as to form two facing sheets joined along one edge. The adhesive may be applied on the interior area adjacent the fold. This provides a folded article that can be readily opened, rupturing the capsules, yet leaves a single artifact rather than two sheets after use.

It is preferred that the capsule-bearing adhesive coated inside portion between the sheets constitute from 60 to 95% of the surface area of the sheets. In two sheet constructions, 10 to 95 percent adhesive coverage can be used to leave an edge or corner that can be readily grasped to pull one sheet form another. Some uses may allow for only a single corner to be uncoated so as to provide a starting point for the separation of the sheets, but the 60 to 95% range is preferred with 70 to 90% more preferred in two sheet constructions.

The pressure-sensitive adhesive usually covers 100% of the back side of the bottom sheet. This is desirable, but not essential. Stripes or other formats of discontinuous pressure-sensitive adhesive could be used.

Any class of adhesives including but not limited to polyurethanes, polyacrylates, polyvinyl resins (e.g., polyvinyl alcohol, polyvinyl chloride), polyamides, polyesters, polyolefins, starches, gum arabic, gelatin and the like may be readily used in the practice of the present invention. Washing of the capsules before mixing them with the adhesive often tends to provide more consistency in their properties by removing low molecular weight, unreacted materials.

In effect, to best practice the present invention it is desirable that certain properties within the article have relative values for each of the materials used. The cohesive strength of the sheet material should exceed the adhesive strength between the binder and the sheet. The adhesive strength of the binder to the sheet should exceed the cohesive strength of the binder and capsules therein. The cohesive strength of the binder should exceed the tensile rupture limits of the capsules. As previously noted, the size of the capsules has an important effect upon the practice of the present invention. With capsules less than 8 $\mu$m, there tends to be loss rupturing of the capsules as to prevent the useful and efficient release of materials. Above 30 $\mu$m, the particles are so large that they are more readily burst by handling of the sheets and manufacturing procedures. Furthermore, with the large size particles it is extremely difficult to control bursting upon separation of the sheets because of increased effects upon adhesive and cohesive properties of materials in contact with the capsules. The preferred ranges of 8 to 30 and 15 to 25 $\mu$m is important to the practice of the present invention. Within these limits, rupture in excess of 50 percent of the capsules can be easily obtained. Rupture in excess of 80 percent of the capsules can often be accomplished in the practice of the present invention within those limits.

The capsules may contain a wide variety of active materials therein. The least useful of materials to be included therein would be coloring agents since separation of the sheets would generally produce uniform coloration rather than a distinct image. The most preferred types of ingredients would be fragrant materials (such as essences and perfumes) or materials which provide chemically active vapors or liquids (e.g., bacteriostats or deodorants) to be wiped on or transferred to another surface. These may or may not also be colored. For example, a testing kit for the presence of chemical vapors could be produced by providing material within the capsules which would react in the vapor phase with the material for which a leak is being investigated. By separating the sheet, rupturing the capsules and exposing the vapor test material, a color forming reaction in the air or on the sheet could be readily observable. Another particularly useful format would be to include the microcapsules within a water-remoistenable adhesive and to use the mixture as the binding adhesive for novelty envelopes. For example, the microcapsules could contain the aromatic essence of baby oil, cake or pizza for invitation envelopes for a baby shower, wedding (or birthday party), or general party, respectively. The sides of the sheets with the capsule-bearing adhesive thereon are preferably printed under the adhesive or adjacent to the adhesive.

This invention may be practiced with a number of various modifications that provide new and useful articles and processes. For example, the adhesive composition with capsules may be associated with various printed formats to form novelty items. The exterior sheets or exposed inner face of the sheets may have questions or stories or rhymes, and under the adhesive may be a printed picture answering the question, depicting the story or completing the rhyme, with the released fragrance emphasizing the picture further.

These and other aspects of the present invention will be shown in the following example.

## Example

A 31.75 kg (70 lb) stock of one side coated paper was used as the top sheet 2 of the label 14. A slurry was prepared from rose fragrance in urea-aldehyde microcapsules manufactured according to the process of Example 10 of U.S. Patent 3,516,941. The slurry contained 64% dry weight of capsules, 24.50% dry weight of poly(vinyl alcohol) (Gelvatol® 40-10), 10.50% poly(vinyl alcohol) (Gelvatol®20-60), and 1% glycerin in water. The slurry was applied to 85% of the surface of the uncoated face of a continuouos web of pressure sensitive label stock at a coating weight of 1.59 kg (3.5 pounds) per $120.77 m^2$ (1300 $ft^2$). After the slurry was applied, the uncoated face of the top sheet was mated to the slurry coated face of the label stock. The laminate was then die-cut into appropriate sizes (e.g., 3 x 8 cm). The cut labels were then removed and affixed to a printed page. The edge of the top sheet which was over the uncoated surface of the label stock was easily raised and grasped by one hand. Upon pulling the top sheet, fragrance was released and the bottom sheet remained firmly adhered to the printed page.

Mechanical handling of the labels by a label applicator did not produce any intolerable level of capsule breakage.

When perforated lines were die-cut into the surface of the top layer, individual strips could be pulled off, leaving the bottom layer adhered to the printed page and leaving additional strips to be pulled off.

## Claims

1. A label for application to a surface, said label comprising
   1) at least two sheets (2,6) or a folded single sheet having opposed faces, the faces of the sheets being bound by an adhesive composition layer (4),
   2) said adhesive composition layer containing microcapsules (12) with a liquid within the shell of the microcapsules,
   3) said microcapsules having an average diameter between 4 and 80 micrometers, and
   4) one of said sheet(s) having a further adhesive (8) on an exterior surface,
   the cohesive strength of said adhesive composition layer being less than the strength of the bond between said adhesive composition and the opposed faces of said sheets, the tensile rupture strength of said microcapsules being such that the cohesive failure of the adhesive results in breakage of the microcapsules, the tensile rupture strength between said two sheets being at least 0.981 $Nm^{-1}$ (1.0g/cm) and less than 88.3 $Nm^{-1}$ (90g/cm) at 20˚ C and 50% relative humidity, the adhesive strength to printed coated paper of said further adhesive on said exterior surface being greater than the cohesive strength of said adhesive composition layer containing microcapsules.

2. A label according to claim 1 wherein said adhesive composition (4) does not extend across the entire surface of the opposed sheet faces and thereby provides a region of non-adherence between said sheet faces.

3. A label according to claim 1 or claim 2 wherein said sheets (2,6) are flexible sheets of coated paper and said adhesive composition (4) comprises said microcapsules (12), a polymeric binder and a viscofier, and said viscofier comprises from 0.25 to 25% by dry weight of said adhesive composition layer.

4. A label according to any preceding claim wherein said folded single sheet or at least two sheets (2,6) are coated paper coated on both faces with a resinous binder and pigment and the adhesive (8) on an exterior surface is a non-pressure-sensitive adhesive, said non-pressure-sensitive adhesive having a strength of adherence to paper which is at least 10% greater than the rupture strength between said opposed faces.

5. A label according to any preceding claim wherein said microcapsules (12) have an average diameter between 8 and 30 micrometers.

6. A label according to any preceding claim wherein said adhesive (8) on an exterior surface is a pressure-sensitive adhesive and has a strippable cover sheet (10) attached thereto.

7. A label according to any preceding claim wherein said microcapsules (12) comprise gelatin and constitute between 20 and 90% by volume of said adhesive composition, said binder comprises between 0 and 78.75% by weight of said adhesive composition and said viscofier comprises between 0.25 and 12% by weight of said adhesive composition.

8. A label according to any preceding claim wherein said microcapsules (12) comprise between 50 and 85% by volume of said adhesive composition and are formed of a urea-aldehyde polymer.

9. A label according to any preceding claim wherein the sheet (2) which is not provided with adhesive on an exterior surface thereof has perforated lines thereon to enable removal of individual portions thereof.

10. A label according to any of claims 1-7 wherein said liquid is an odor releasing material.

11. A label according to any of claims 1-7 wherein an image that appears on the interior surface of said one sheet (6) is different from an image that appears on the outer surface of the other sheet (2).

12. A label according to any preceding claim which comprises two discrete sheets (2,6) bound by said adhesive composition layer (4).

**Revendications**

1. Etiquette applicable sur une surface donnée, ladite étiquette comprenant :
   1) au moins deux feuilles (2,6) ou une seule feuille pliée présentant des faces opposées, les faces des feuilles étant liées à l'aide d'une couche de produit adhésif (4),
   2) ladite couche de produit adhésif contenant des microcapsules (12) renfermant dans leur enveloppe un produit liquide,
   3) lesdites microcapsules possédant un diamètre moyen de 4 à 80 micromètres, et
   4) l'une desdites feuille(s) présentant sur la face extérieure un autre adhésif (8),
   la force cohésive de ladite couche de produit adhésif étant inférieure à la résistance de la liaison entre ledit produit adhésif et les faces opposées desdites feuilles, la résistance à la rupture par traction desdites microcapsules étant telle que la rupture cohésive de l'adhésif entraîne l'éclatement des microcapsules, la résistance à la rupture par traction observée entre lesdites deux feuilles s'élèvant au moins à 0,981 Nm⁻¹ (1,0g/cm) en restant inférieure à 88,3 Nm⁻¹ (90g/cm) à une température de 20° C pour un taux d'humidité relative de 50 %, la force adhésive s'exerçant sur le papier couché imprimé dudit autre adhésif déposé sur ladite face extérieure étant supérieure à la force cohésive de ladite couche de produit adhésif contenant les microcapsules.

2. Etiquette selon la revendication 1 caractérisée en ce que ladite composition adhésive (4) ne couvre pas la totalité des faces opposées de la feuille et présente ainsi une zone de non-adhérence entre lesdites faces de la feuille.

3. Etiquette selon les revendications 1 ou 2, caractérisée en ce que lesdites feuilles (2,6) sont des feuilles souples de papier couché et caractérisée en ce que ledit produit adhésif (4) renferme lesdites microcapsules (12), un liant polymère et un épaississant, ce dernier entrant pour 0,25 à 25 % du poids sec de ladite couche de produit adhésif.

4. Etiquette selon l'une quelconque des revendications précédentes, caractérisée en ce que la feuille simple pliée en deux ou les deux feuilles au moins (2,6) sont en papier couché, enduit sur les deux faces d'un liant composé d'une résine, de pigments, et l'adhésif (8) déposé sur la surface extérieure n'est pas auto-collant, ledit adhésif possédant une force d'adhérence au papier de 10 % au moins supérieure à la résistance à la rupture entre lesdits côtés opposés.

5. Etiquette selon l'une quelconque des revendications précédentes, caractérisée en ce que lesdites microcapsules (12) possèdent un diamètre moyen allant de 8 à 30 micromètres.

8

**6.** Etiquette selon l'une quelconque des revendications précédentes, caractérisée en ce que ledit adhésif (8) déposé sur une surface externe est auto-collant et se trouve sous une feuille de protection pelable (10).

**7.** Etiquette selon l'une quelconque des revendications précédentes, caractérisée en ce que lesdites microcapsules (12) contiennent de la gélatine et représentent de 20 à 90 % du volume dudit produit adhésif, ledit liant s'élèvant de 0 à 78,75 % du poids dudit produit adhésif et ledit épaississant comptant pour 0,25 à 12 % du poids dudit produit adhésif.

**8.** Etiquette selon l'une quelconque des revendications précédentes, caractérisée en ce que lesdites capsules représentent de 50 à 85 % du volume dudit adhésif et proviennent d'un polymère urée-aldéhyde.

**9.** Etiquette selon l'une quelconque des revendications précédentes, caractérisée en ce que celle des feuilles (2) dont la face externe n'a pas été enduite d'adhésif présente des lignes perforées permettant d'en enlever des parties séparément.

**10.** Etiquette selon l'une quelconque des revendications de 1 à 7, caractérisée en ce que ledit élément liquide est un matériau libérant une odeur.

**11.** Etiquette selon l'une quelconque des revendications de 1 à 7, caractérisée en ce qu'une image apparaîssant sur la face interne de ladite feuille simple (6) diffère d'une image qui apparaît sur la face externe de l'autre feuille (2).

**12.** Etiquette selon l'une quelconque des revendications précédentes, caractérisée en ce qu'elle est constituée de deux feuilles distinctes (2,6) liées par ladite couche de produit adhésif (4).

## Ansprüche

**1.** Etikett zum Anbringen an einer Oberfläche mit
1) mindestens zwei Blättern (2, 6) oder einem gefalteten einzigen Blatt, mit zwei einander gegenüberliegenden Flächen, die durch eine Schicht (4) aus einer Klebstoffzusammensetzung stoffschlüssig miteinander verbunden sind,
2) wobei die Schicht aus der Klebstoffzusammensetzung Mikrokapseln (12) enthält, die in einer Schale der Mikrokapseln eine Flüssigkeit enthalten,
3) die Mikrokapseln einen durchschnittlichen Durchmesser zwischen 4 und 80 $\mu$m haben, und
4) das Blatt oder eines der Blätter auf einer Außenfläche mit einem weiteren Klebstoff (8) versehen ist, wobei die Kohäsionsfestigkeit der Schicht aus der Klebstoffzusammensetzung geringer ist als die Festigkeit der stoffschlüssigen Verbindung zwischen der Klebstoffzusammensetzung und den einander gegenüberliegenden Flächen der Blätter, die Mikrokapseln eine solche Reißfestigkeit haben, daß ein Auseinanderreißen des Klebstoffes zum Bruch der Mikrokapseln führt, die Reißfestigkeit zwischen den beiden Blättern bei 20° C und einer relativen Luftfeuchtigkeit von 50%, mindestens 0,981 Nm$^{-1}$ (1,0 g/cm) beträgt und niedriger ist als 88,3 Nm$^{-1}$ (90 g/cm) und die Haftfestigkeit zwischen dem auf der Außenfläche vorgesehenen, weiteren Klebstoff und bedrucktem gestrichenem Papier größer ist als die Kohäsionsfestigkeit der Mikrokapseln enthaltenden Schicht aus der Klebstoffzusammensetzung.

**2.** Etikett nach Anspruch 1, dadurch gekennzeichnet, daß sich die Klebstoffzusammensetzung (4) nicht über die ganze Fläche der einander gegenüberliegenden Blattflächen erstreckt, so daß zwischen den Blattflächen ein adhäsionfreier Bereich vorhanden ist.

**3.** Etikett nach Anspruch 1 oder 2, dadurch gekennzeichnet, daß die Blätter (2, 6) flexible Blätter aus gestrichenem Papier sind und daß die Klebstoffzusammensetzung (4) die Mikrokapseln (4) ein polymeres Bindemittel und ein viskositäterhöhendes Mittel enthält, das 0,25 bis 25% des Trockengewichtsder Schicht aus der Klebstoffzusammensetzung ausmacht.

**4.** Etikett nach einem der vorhergehenden Ansprüche, dadurch gekennzeichnet, daß das gefaltete einzige

9

Blatt oder die mindestens zwei Blätter (2, 6) aus gestrichenem Papier bestehen, das auf beiden Flächen mit einem Harzbindemittel und Pigment überzogen ist, und daß der auf einer Außenfläche vorgesehene klebstoff (8) ein Klebstoff ohne Haftklebeeigenschaften ist, dessen Haftfestigkeit an Papier minderstens 10% höher ist als die Reißfestigkeit zwischen den beiden einander gegenüberliegenden Flächen.

5. Etikett nach einem der vorhergehenden Ansprüche, dadurch gekennzeichnet, daß der durchschnittliche Durchmesser der Mikrokapseln (12) zwischen 8 und 30 $\mu$m liegt.

6. Etikett nach einem der vorhergehenden Ansprüche, dadurch gekennzeichnet, daß der auf einer Außenfläche vorgesehene Klebstoff (8) ein Haftkleber ist und daß an ihm ein abziehbares Deckblatt (10) angebracht ist.

7. Etikett nach einem der vorhergehenden Ansprüche, dadurch gekennzeichnet, daß die Mikrokapseln mindestens teilweise aus Gelatine bestehen und daß die Klebstoffzusammensetzung zu 20 bis 90 Vol.-% aus den Mikrokapseln, zu 9 bis 78,75 Gew.-% aus dem Bindemittel und zu 0,25 bis 12 Gew.-% aus dem viskositäterhöhenden Mittel besteht.

8. Etikett nach einem der vorhergehenden Ansprüche, dadurch gekennzeichnet, daß die Klebstoffzusammensetzung zu 50 bis 85 Vol.-% aus den Mikrokapseln (12) besteht und diese aus einem Hartstoff-Aldehyd-Polymer bestehen.

9. Etikett nach einem der vorhergehenden Ansprüche, dadurch gekennzeichnet, daß jenes Platt, das auf einer Außenflächenicht mit Klebstoff versehen ist, mit Perforationslinien versehen ist, die ein Abtrennen von einzelnen Teilen dieses Blattes ermöglichen.

10. Etikett nach einem der Ansprüche 1 bis 7, dadurch Gekennzeichnet, daß die Flüssigkeit eine Duftstoff abgebende Substanz ist.

11. Etikett nach einem der Ansprüche 1 bis 7, dadurch gekennzeichnet, daß auf der Innenfläche des genannten einen Blattes (6) ein anderes Bild erscheint als auf der Außenfläche des anderen Blattes (2).

12. Etikett nach einem der vorhergehenden Ansprüche mit zwei diskreten Blättern, die durch die Schicht (4) aus der Klebstoffzusammensetzung miteinander verbunden sind.